# EUROPÄISCHE PATENTSCHRIFT

(11) **EP 0 789 532 B1**
(45) Veröffentlichungstag und Bekanntmachung des Hinweises auf die Patenterteilung: **12.08.1998**
(21) Anmeldenummer: 95936575.0
(22) Anmeldetag: 31.10.1995
(51) Int. Cl.: A61B 5/14, B01L 3/00

(54) **PROBENABNAHMEGERÄT**
SAMPLE-TAKING DEVICE
DISPOSITIF DE PRELEVEMENT D'ECHANTILLONS

(30) Priorität: 03.11.1994 DE 9417612 U
(43) Veröffentlichungstag der Anmeldung: 20.08.1997
(73) Patentinhaber: KLOTH, Bernd, 22399 Hamburg (DE)
(72) Erfinder: KLOTH, Bernd, 22399 Hamburg (DE)
(74) Vertreter: Gerbaulet, Hannes
(86) Internationale Anmeldenummer: EP9504269
(87) Internationale Veröffentlichungsnummer: WO9614017

(56) Entgegenhaltungen:
- EP-A- 0 421 175
- WO-A-79/01131
- DE-A- 2 558 311
- PATENT ABSTRACTS OF JAPAN vol. 12 no. 307 (P-747) ,22.August 1988 & JP,A,63 078043 (MATSUSHITA ELECTRIC WORKS LTD) 8.April 1988,

## Beschreibung

Das erfindungsgemäße Probenabnahmegerät ist überall dort einsetzbar, wo mittels einer Kapillare eine Flüssigkeit entnommen werden soll.

Die Erfindung betrifft ein Probenabnahmegerät zur Entnahme von Kapillarblut direkt am Patienten oder zur Entnahme einer Probe aus einer Flüssigkeit mittels einer Kapillare.

### Stand der Technik

In der klinischen Chemie müssen vielfach Kapillarblutentnahmen durchgeführt werden. Dazu wird eine Fingerbeere, das Ohrläppchen oder bei Säuglingen und Kleinkindern die Ferse mit einer Lanzette punktiert. Der erste Blutstropfen wird, da Gewebethrombokinase mit austritt, enfernt und verworfen, um eine nachfolgende Analyse nicht zu verfälschen.

Das anschließend austretende Blut wird mittels einer Pipette entnommen. Bei dieser Methode ist es eine Pipette, die über einen Schlauch und ein Mundstück zum Ansaugen des Blutes durch die blutabnehmende Person verfügt. Anschließend wird das Blut durch Ausblasen der Pipette in eine Küvette überführt. Dieses Verfahren birgt jedoch zahlreiche Fehlerquellen, da das Volumen der Probenflüssigkeit nicht mit der erforderlichen Genauigkeit abgemessen werden kann. Dies ist bedingt durch die direkte Verbindung der Luftsäule über dem Blut in der Pipette mit der Abnahmeperson. Außerdem ist es nicht auszuschließen, daß die Probe verunreinigt wird durch die Atemluft, die Feuchtigkeit und Keime mitführt. Auch umgekehrt besteht Infektionsgefahr für die blutabnehmende Person, wenn das Blut versehentlich aus der Pipette durch den Schlauch in den Mund gelangt. Zudem ist diese Methode nicht für automatisierte Analysenverfahren geeignet, da ein definierter Zeitpunkt zum Starten bei Ausblasen des Blutes nicht erhalten werden kann. Durch das Ausblasen kann auch eine starke Blasen und/oder Schaumbildung einsetzen, was die Meßergebnisse ebenfalls stark verfälscht.

Um diese Fehler zumindest teilweise zu beheben, ist gemäß der DE-AS 24 22 260 vorgeschlagen worden, mit End-Zu-Endkapillaren zu arbeiten, wobei hier über die Länge und den Querschnitt der Kapillare das Probenvolumen definiert ist. Die Kapillare wird unter einem geeigneten Winkel an dem Blutstropfen herangeführt, worauf sich die Kapillare aufgrund des Kapillareffekts automatisch bis zum Kapillarende vollsaugt. Die Kapillare wird mit dem darin enthaltenen Blut in eine Küvette verbracht und diese mit einem Stopfen verschlossen. Ein Durchmischen der Probe erfolgt durch vorsichtige Kippbewegungen. Diese Methode ermöglicht eine kontaminationslose Probenbereitung mit definerten Volumen.

Obwohl in o.g. Druckschrift angestrebt wird, daß die Kapillare während der Messung innen an der Küvettenwandung haften soll, hat sich in der Praxis gezeigt, daß die Kapillare oftmals sich doch im Meßkanal befindet und die Messung massiv stört. Dies geschieht unter anderem aus Unachtsamkeit des Bedienungspersonals und auch durch eine fehlerhafte Kapillare, die durch eine Unregelmäßigkeit der Oberfläche nicht an der Wandung haften kann. Auch bei dieser Methode ist ein definierter Startzeitpunkt für ein automatisiertes Meßverfahren nicht einzuhalten, so daß nur ein eingeschränkter Einsatzbereich zur Verfügung steht.

Aus der WO-A-79/01131 ist ein Probenabnahmegerät zur Entnahme von Kapillarblut bekannt, wobei das Gerät eine Kapillare und einen die Kapillare haltenden Kapillarhalter aufweist. Der die Kapillare haltende Kapillarhalter ist mit einer Küvette verbindbar oder an der Küvette angeordnet. Dabei ist der Kapillarhalter mit der Kapillare in der Küvette verschiebbar.

Der Kapillarhalter hat einen Fortsatz, durch den sich eine Durchgangsbohrung erstreckt und auf den eine Druckkappe aufsteckbar ist. In dem Kapillarhalter sind Rillen angebracht und in der Küvette ist eine Reagenzflüssigkeit.

Bei dem aus der EP 0 421 175 bekannten Kapillarröhrchen ist bereits eine Druckkappe vorgesehen, die dazu dient, die Probenflüssigkeit aus der Kapillare zu drücken.

### Aufgabe, Lösung, Vorteile

Es ist daher Aufgabe der Erfindung, ein Probenabnahmegerät zu schaffen, daß es ermöglicht, Analysen der Probe frei von Volumenfehlern im Probenbereich und kontaminationslos durchzuführen. Zusätzlich soll ein definierter Startzeitpunkt für einen automatischen Reaktionsstart gegeben sein.

Diese Aufgabe wird durch die in Anspruch 1 gekennzeichneten Merkmale gelöst.

Die Kapillare wird dabei direkt in der für die Untersuchung vorgesehenen Küvette gehalten, so daß die Probenflüssigkeit insbesondere Blut, mit der Kapillare als definiertes Probenvolumen aufgenommen und anschließend zum Analysenstart in die Küvette überführt wird.

Es ist dabei so, daß eine mit einem Reagenz und auch mit einem Rührkörper in Form eines Stäbchens oder einer Kugel versehen ist und mittels eines aufsteckbaren oder aufschraubbaren Küvettenverschlusses verschlossen ist. Anstelle des Verschlusses kann auch eine Folie vorgesehen werden, die am Küvettenrand fest haftet.

Vor Durchführung der Untersuchung wird dann der Verschluß entfernt bzw. die Folie abgezogen. Die Küvette wird anschließend sofort mit dem Kapillarhalter, der vorteilhafterweise als Stopfen für die Küvette ausgebildet ist, versehen. Dazu wird eine Küvette mit einem Kapillarhalter ausgestattet, der die Kapillare derart in der Küvettenöffnung hält, daß diese nicht in die Reagenzien eintaucht.

In dem Kapillarhalter ist die Kapillare bevorzugterweise so angeordnet, daß sie mit ihrem einen Ende ein Stück aus dem Kapillarhalter herausragt, um eine problemlose Probenaufnahme zu ermöglichen. Die Kapillare wird hierzu mit der anhängenden Küvette an die Probe herangeführt, so daß das Probenvolumen von der Kapillare aufgenommen wird.

Es kann vorgesehen werden, daß die Küvette mit der Kapillare vor Aufnahme der Probe in eine Heizvorrichtung, beispielsweise in das Analysengerät eingesetzt wird, um eine Vorwärmung, beispielsweise auf 37 Grad Celsius durchzuführen.

Sowohl mit aufgenommener Probe als auch vor Probenaufnahme ist die Kapillare-Küvetten-Kombination völlig handhabungssicher, da ein Auslaufen von Reagenz aus der Küvette durch den Verschluß und ein Auslaufen von Probenflüssigkeit durch die Kapillarkräfte sicher verhindert wird. Außerdem ist vorgesehen, daß das andere Ende der Kapillare ein Stück in die Küvette hineinragt, und zwar nur soweit, daß das Kapillarenende die Oberfläche einer eingefüllten Reagenz nicht berührt und einen Abstand hierzu aufweist. So wird auch sicher verhindert, daß Reagenzflüssigkeit in die Kapillare eindringt.

Zum Start der vorgesehenen Analyse wird die Probenflüssigkeit aus der Kapillare in die Küvette verbracht. Hierzu kann vorgesehen werden, daß die von der Küvette wegweisende Öffnung der Kapillare mittels einer Vorrichtung, die Bestandteil oder Zusatzteil des Blutabnahmegeräts ist, mit einem komprimierten Gas derart beaufschlagbar ist, daß zwischen der von der Küvette wegweisenden Öffnung und der zur Küvette hinweisenden Öffnung der Kapillaren eine definierte Druckdifferenz herrscht, wobei diese Druckdifferenz derart bemessen ist, daß mit Entleerung der Probenflüssigkeit in die Küvette durch die Druckbeaufschlagung die Druckdifferenz ausgeglichen ist oder ggf. noch eine geringe Menge Gas ebenfalls austritt. Hierdurch wird ein genau definiertes Probenvolumen kontaminationsfrei in die Küvette gebracht. Es ist vorgesehen, daß das Volumen in der Druckkappe derart bemessen ist, daß beim Aufsetzen der Schutzkappe auf den Fortsatz und anschließenden vollständigen Aufschieben die Probenflüssigkeit aus der Kapillare gerade herausgedrückt wird. So kann durch einfaches Aufsetzen und Herunterdrücken der Druckkappe die Entleerung der Kapillare bzw. die Füllung der Küvette erfolgen. Dazu ist vorteilhafterweise vorgesehen, ein Blutabnahmegerät mit integrierter Küvette zu Analysenzwecken, wobei die Küvette Reagenzflüssigkeiten enthält, und mit einer Kapillare, die mit zu untersuchender Probenflüssigkeit füllbar ist, mit einer Druck- und Schutzkappe zu versehen, mittels derer ein definiertes Luft-Volumen derart durch die Kapillare gepreßt weden kann, daß die Probenflüssigkeit bzw. das Blut ohne Blasen- oder Schaumbildung in die Küvette austreten kann.

Die Kapillare mit Halter kann auch ohne Verbindung mit der Küvette zur Probenentnahme benutzt werden. Die so gefüllte Kapillare wird dann auf die mit Reagenz beschichtete Küvette gesetzt. Der Kapillarhalter an der aufsteckbaren Seite zur Küvette muß so ausgeführt sein, daß bei Aufsetzen des Halters die Luft seitlich zwischen Küvettenrand und Halter entweichen kann. Somit verbleibt alle Flüssigkeit in der Kapillare, anschließend wird der Startvorgang wie beschrieben mit der Druckkappe erfolgen.

Bei dem Herunterdrücken der Druckkappe wird das freie Ende der Kapillare ebenfalls heruntergedrückt, und zwar so, daß daß Ende ganz in die Durchgangsbohrung des Kapillarhalters bzw. des Fortsatzes hineingeschoben wird. Hierdurch wird gleichzeitig das andere Ende weiter hinausgedrückt, so daß es jetzt bis in die Reagenz- bzw. Probenflüssigkeit reicht.

Bei einem anschließenden Rührvorgang werden die am Ende der Kapillare innen und außen möglicherweise noch anhaftenen Probenreste abgespült, so daß mit völliger Sicherheit erreicht wird, daß das vorgesehene Probenvolumen in die Küvette gelangt und mit dem Reagenz vermischt wird.

Für spezielle Zwecke kann vorgesehen werden, daß die Kapillare innen mit einer Antikoagulanz beschichtet ist, so daß eine aufgenommene Blutprobe nicht koaguliert. So wird es ermöglicht, daß die Probenentnahme und die Analyse zeitlich versetzt durchgeführt werden können.

Im Zentrum des Kapillarhalters befindet sich ein zylindrischer Fortsatz, in dem die Kapillare in einer Durchgangsbohrung steckt. Auf diesen Fortsatz wird die Schutz-und Druckkappe aufgebracht, wobei der Fortsatz und die herausragende Kapillare in den Innenraum der Schutzkappe hineinragen. Durch Bewegung der Schutzkappe in Richtung des Kapillarhalters bzw. der Küvette wird die in der Schutzkappe verbliebende Luft komprimiert, da sich der Fortsatz und der Innenraum der Schutzkappe wie Pumpenkolben und Kolbengehäuse verhalten. Dadurch liegt auf der Kapillaröffnung in der Bohrung der Schutzkappe ein definierter Druck, wodurch das Blut aus der Kapillare in die Küvette getrieben wird und zwar derart, daß lediglich die Probenflüssigkeit und evtl. eine geringfügige Menge Luft aus der Kapillare in die Küvette gelangen. Die evtl. mit austretende Menge (Volumen) an Luft muß so bemessen sein, daß keine Blasen- und/oder Schaumbildung auftritt. Dies ist nach einer bevorzugten Ausführungsform 1/10 - 1/2 des Volumens der Kapillare.

Vorteilhafterweise ist der Kapillarhalter zur Küvettenseite an den Auflageflächen zum Küvettenrand hin mit Rillen versehen, durch die Luft aus der Küvette austreten kann, so daß in der Küvette sich kein Überdruck aufbauen kann. Die Küvette kann beliebig ausgeformt sein, wie es den Anforderungen, die durch den Analysenapparat bestimmt werden, entspricht.

Es ist ebenfalls vorteilhaft nach einer bestimmten Ausführungsform vorgesehen, die Küvette mit einer Folie, einem Drehverschluß oder einem Verschlußstopfen verdunstungsdicht zu verschließen. Fehler durch Verdunstung der Reagenzien bei längerem Stehen können dadurch vermieden werden. Beim Aufsetzen des Kapillarhalters wird der Verschluß oder die Folie enfernt oder zerstört.

Eine weitere Vorgehensweise ist, die Küvette mit einer definierten Menge eines Antikoagulanz-Puffergemisches in diesem Fall ein Citrat Puffer zu befüllen und die Probe wie beschrieben in diesem zu verbringen. Damit kann die Probe bis zu 24 Stunden stabil gehalten werden und somit auch an einem anderen Ort als dem der Blutabnahme gemessen werden. Um einen Reaktionsstart auszulösen wird die Kapillare von der Küvette entfernt und das Startreagenz mittels einer Pipette der Probe zugeführt.

Durch die erfindungsgemäße Ausgestaltung mit Kapillarhalter und Schutzkappe kann nach durchgeführter Analyse die Probe samt Küvette dicht verschlossen in den Müll entsorgt werden, ohne daß eine Infektionsgefahr besteht.

Das erfindungsgemäße Probenabnahmegerät eignet sich daher auch besonders für automatische Analysen, die von einen medizinischen Laien, beispielsweise einen Patienten, der ein Medikament zur Hemmung der Blutgerinnung nehmen muß, durchgeführt werden kann. Es kann jedoch jede Art von flüssigen Proben für die unterschiedlichsten Analysen abgenommen und einer Analyse zugeführt werden.

Das Probenabnahmegerät wird dann wie folgt gebraucht:

Zur Blutentnahme wird die Kapillar-Küvetten-Kombination an den Blutstropfen unter einem geeigneten Winkel gehalten, worauf sich die Kapillare automatisch füllt. Anschließend wird die Kapillar-Küvetten-Kombination in den Meßkanal des Analysators gebracht. Durch Aufsetzen der Schutzkappe wird Luft durch die Kapillare gepreßt und das Blut tritt in die mit Reagenzien gefüllte Küvette ein. Durch das Eintreten des Blutes in die Reagenzien kann je nach Analysengerät die Messung automatisch gestartet werden und die gewünschten Meßdaten, wie z.B. die Gerinnungszeit ermittelt werden.

Weiterhin vorteilhafte Ausgestaltungen sind in den Unteransprüchen gekennzeichnet.

### Kurze Beschreibung der Zeichnungen

Nachstehend wird die Erfindung anhand von Zeichnungen erläutert. Es zeigen
- Fig. 1: in einer senkrechten Schnittdarstellung das erfindungsgemäße Probenabnahmegerät,
- Fig. 2: in einer schaubildlichen Ansicht eine Blutabnahme mit dem Probenabnahmegerät gemäß Fig.1,
- Fig. 3: in einer Explosionsdarstellung das Probenabnahmegerät gemäß Fig. 1 mit einem vor Aufsetzen des Kapillarhalters zu entfernenden Küvettenverschluß,
- Fig. 4: in einer senkrechten Schnittdarstellung das Probenabnahmegerät mit in einer ersten Stellung aufgesetzter Druck- und Schutzkappe,
- Fig. 5: in einer schaubildlichen Ansicht ein für das Probenabnahmegerät vorgesehenes Analysengerät und
- Fig. 6 - 8: in schematischen Darstellungen die Handhabung des Probenabnahmegerätes mit einem Analysengerät.

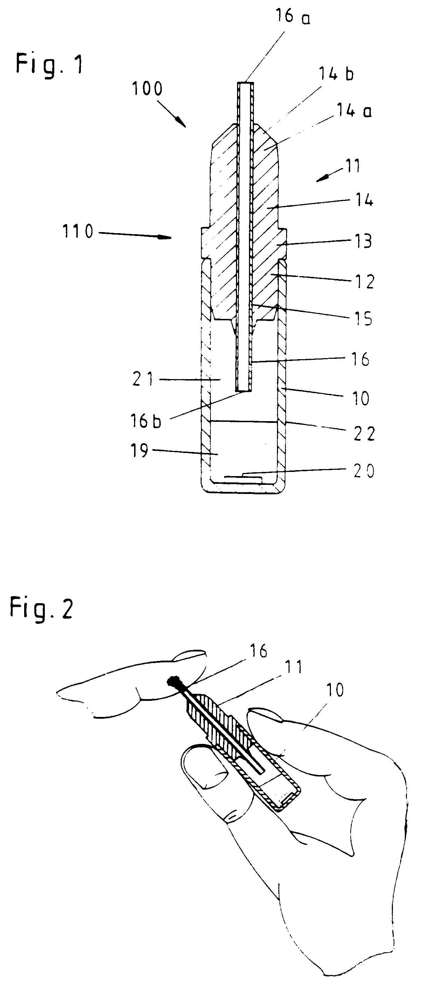

Das in Fig. 1 bis 8 dargestellte Blutentnahmegerät 100 weist eine Küvette 10 auf, die mit einem Kapillarhalter 11 verschlossen ist. Dieser Kapillarhalter 11 weist zur Küvette 10 hin einen stopfenartigen Bereich 12 auf, der in die Küvette 10 hineinreicht und damit den Verschluß bewirkt.

Von außen umgreift der Kapillarhalter 11 die Küvette 10 mittels eines umlaufenden Randes 13, der an der Außenseite 22 der Küvette 10 heruntergezogen sein kann. Am Kapillarhalter 11 dem stopfenartigen Bereich 12 gegenüberliegend befindet sich ein Fortsatz 14, durch den, wie auch durch den stopfenförmigen Bereich 12 eine Durchgangsbohrung 15 verläuft, in die die Kapillare 16 gesteckt ist. Auf den Fortsatz 14 ist eine Schutz- und Druckkappe 17 aufsetzbar. Hierzu ist an dem Fortsatz 14 am freien Ende 14a eine umlaufende Fase 14b ausgebildet, um das Aufsetzen der Kappe 17 auf den Fortsatz 14 zu erleichtern.

Der Bereich der Schutzkappe 17, in den der Fortsatz 14 hineinragt, weist einen dem Außendurchmesser A des Fortsatzes 14 entsprechenden Innendurchmesser I auf, so daß der Fortsatz 14 in der Schutzkappe 17 wie ein Pumpenkolben in einem Pumpengehäuse verschiebbar ist. Das Innenvolumen 23 der Schutzkappe 17 kann über die Variation der Tiefe derart bemessen werden, daß die nach Aufstecken der Schutzkappe 17 im Innenvolumen 23 verbliebene Luft gerade ausreicht, um die Probe in die Küvette 10 auszutreiben. Die in der Schutzkappe 17 verbliebene Luft wird dabei komprimiert, so daß im Innenraum der Schutzkappe ein höherer Druck herrscht als in der Umgebung bzw. in der Küvette 10.

Die von der Küvette 10 wegweisende Öffnung 16a der Kapillare 16 ist demnach mit Druck beaufschlagt, so daß zwischen der von der Küvette 10 wegweisenden Öffnung 16a und der zur Küvette 10 hinweisenden Öffnung 16b der Kapillaren 16 eine Druckdifferenz besteht. Diese Druckdifferenz wird durch geeignete Auswahl des Volumens der Schutz- und Druckkappe 17 definiert, und zwar derart, daß nach Entleerung der Probenflüssigkeit in die Küvette 10 durch die Druckbeaufschlagung der Öffnung 16a die Druckdifferenz gerade ausgeglichen ist bzw. noch eine geringe Menge Luft ebenfalls austritt. Diese Menge an Luft darf jedoch nur so groß sein, daß keine Blasen- und/oder Schaumbildung auftritt. In der Küvette 10 befindet sich Reagenzflüssigkeit 19 sowie ein Rührkörper 20.

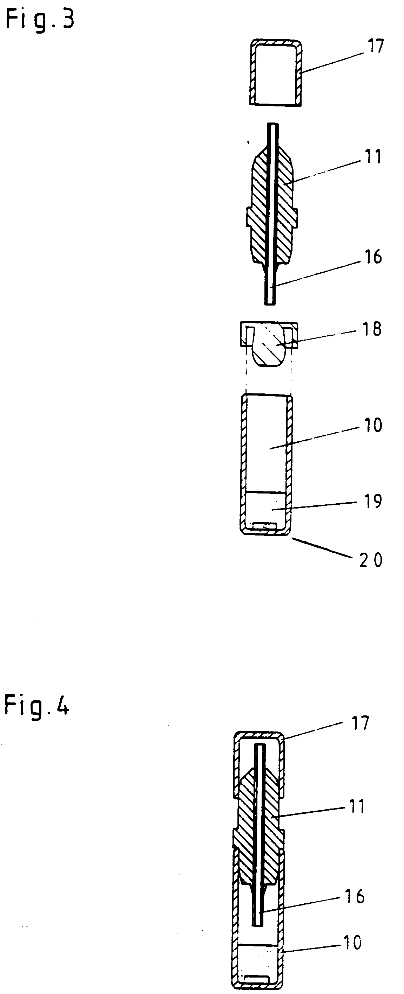

In Fig. 2 ist dargestellt, wie das erfindungsgemäß aus der Küvette 10, dem Kapillarhalter 11 und der Kapillare 16 bestehende Probenabnahmegerät benutzt wird, um eine aus einer Fingerbeere austretende Blutprobe abzunehmen. Hierzu ist - wie in Fig. 3 angedeutet - zunächst der Küvettenverschluß 18 abgenommen und anschließend der Kapillarhalter 11 mit der von ihm gehalterten Kapillare 16 aufgesetzt worden.

Es ist auch möglich, daß die Druckkappe 17 durch den Küvetten-Verschlußstopfen 18 in seiner Funktion ersetzt werden kann, der so im Innenraum ausgeführt sein muß, wie es für die Druckkappe 17 beschrieben ist.

Nach dem Abnehmen der Probe wird die Druckkappe 17 in eine erste, in Fig. 4 dargestellte Stellung aufgesetzt. Die dabei in der Druckkappe 17 bewirkte erste Druckerhöhung steht noch im Gleichgewicht mit den Kapillarkräften in der Kapillare, so daß aus dieser die in der Zeichnung nicht dargestellte, in Fig. 4 in der Kapillare befindliche Probe noch nicht austritt. Es kann dann problemlos ein Transport zu einer Analyse durchgeführt werden.

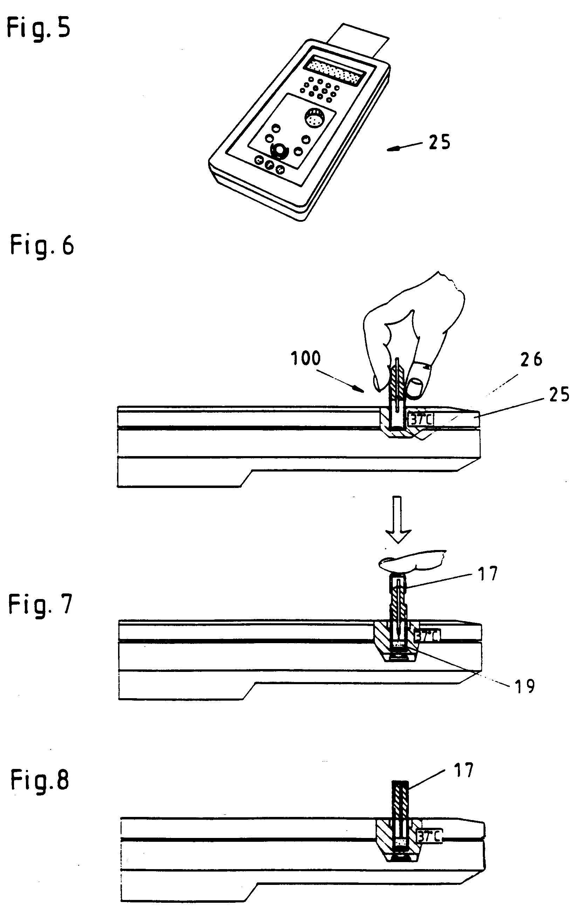

Ein besonders für medizinisch nicht vorgebildete Personen geeignetes Analysengerät 25 ist in Fig. 5 dargestellt. In diesem kann das zur Probenabnahme vorbereitete Gerät vorbereitet werden, indem es in den Untersuchungskanal 26 eingesetzt und dort auf die erforderliche Temperatur, z.B. 37 Grad Celsius erwärmt wird (Fig. 6).

Nach Probenabnahme und Ansetzen der Schutz- und Druckkappe 17 gemäß Fig. 2 und Fig. 4 wird das Probenabnahmegerät 100 in das Analysegerät 25 eingesetzt. Hier wird durch Runterdrücken der Schutz- und Druckkappe 17 die Probe, z.B. Kapillarblut, in die Küvette 10 bzw. in die Reagenzflüssigkeit 19 gedrückt. Durch die Änderung der Lichtdurchlässigkeit der Reagenzflüssigkeit 19 durch das zugeführte Blut bzw. die zugeführte Probe wird automatisch der Analysenvorgang gestartet (Fig. 8), was zu immer gleichen Untersuchungsbedingungen führt. Es kann bevorzugterweise vorgesehen werden, daß das Analysengerät 25 die Ergebnisse der Untersuchung in einem internen Speicher speichert.

### Bezugszeichenliste

- Probenabnahmegerät: 100
- Halterungseinrichtung: 110
- Küvette: 10
- Kapillarhalter: 11
- stopfenartiger Bereich: 12
- Rand: 13
- Fortsatz: 14
- freies Ende: 14 a
- Fase: 14 b
- Durchgangsbohrung: 15
- Kapillare: 16
- Öffnungen der Kapillare: 16a, 16b
- Schutzkappe: 17
- Küvettenverschluß: 18
- Reagenzflüssigkeit: 19
- Rührkörper: 20
- Innenraum: 21
- Außenseite: 22
- Innenvolumen: 23
- Analysengerät: 25
- Untersuchungskanal: 26

## Patentansprüche

1. Probenabnahmegerät (100) zur Entnahme von Kapillarblut direkt am Patienten oder zur Entnahme einer Probe aus aus einer Flüssigkeit mittels einer Kapillare (16), welches Probenabnahmegerät eine Kapillare und eine Küvette aufweist, wobei die Kapillare (16) zur Aufnahme von zu untersuchender Probenflüssigkeit mittels einer Halterungseinrichtung (110) mit der Küvette (10) verbindbar ist und die Küvette (10) als Halterungseinrichtung (110) einen die Kapillare (16) haltenden Kapillarhalter (11) aufweist,
dadurch gekennzeichnet,
daß der Kapillarhalter (11) einen stopfenartigen Bereich (12), der im Gebrauch in die Küvette (10) hineinreicht, aufweist, wobei der Kapillarhalter (11) einen umlaufenden Rand (13) besitzt, der größer ist als der Küvettenumfang, und daß auf der von der Küvette (10) wegweisenden Seite im Zentrum des Kapillarhalters (11) ein Fortsatz (14) vorgesehen ist, durch den sich, wie auch durch den stopfenartigen Bereich (12) eine Durchgangsbohrung (15) erstreckt, in die die Kapillare (16) gesteckt ist und daß eine Druckkappe (17) vorgesehen ist, die auf den Fortsatz (14) aufsteckbar ist, wobei das Volumen (23) in der Druckkappe (17) derart bemessen ist, daß beim Aufsetzen der Druckkappe (17) auf den Fortsatz (14) und anschließenden vollständigen Aufschieben die Probenflüssigkeit aus der Kapillare (16) gerade herausgedrückt wird, daß in einer Reagenzflüssigkeit (19) in der Küvette (10) ein Antikoagulanz, wie z.B. ein Citratpuffer enthalten ist und daß in der Küvette (10) ein Rührkörper (20) enthalten ist.

2. Probenabnahmegerät nach Anspruch 1,
dadurch gekennzeichnet,
daß die Kapillare (16) in dem Kapillarhalter (11) längsverschieblich gehaltert ist.

3. Probenabnahmegerät nach Anspruch 1 oder 2,
dadurch gekennzeichnet,
daß die Küvette (10) mit dem Kapillarhalter (11) verschlossen ist.

4. Probenabnahmegerät nach Anspruch 1,
dadurch gekennzeichnet,
daß die Druckkappe (17) in eine erste, den Fortsatz (14) nur teilweise übergreifenden Stellung aufsetzbar und in eine zweite, den Fortsatz (14) vollständig übergreifende Stellung aufschiebbar ausgebildet ist.

5. Probenabnahmegerät nach einem der Ansprüche 1 bis 4,
dadurch gekennzeichnet,
daß die Durchgangsbohrung (15) in dem Kapillarhalter (11) kürzer als die Länge der Kapillare (16), insbesondere 20 % bis 30 % kürzer ist.

6. Probenabnahmegerät nach Anspruch 1,
dadurch gekennzeichnet,
daß im Anlagebereich des Kapillarhalters (11) an der Küvette (10) und/oder im Auflagebereich des umlaufenden Randes (13) des Kapillarhalters (11) zur Küvette (10) hin Rillen eingebracht sind, die einen Druckausgleich zwischen dem Innenraum (21) der Küvette (10) und der Umgebung erlauben.

7. Probenabnahmegerät nach Anspruch 1 oder 6,
dadurch gekennzeichnet,
daß der umlaufende Rand (13) des Kapillarhalters (11) in Richtung der Küvette (10) an deren Außenseite (22) herabgezogen ist.

8. Probenabnahmegerät nach einem der Ansprüche 1 bis 7,
dadurch gekennzeichnet,
daß die Küvette (10) in getrenntem Zustand von Kapillarhalter (11) mittels einer verdunstungsdichten Folie verschlossen ist.

9. Probenabnahmegerät nach einem der Ansprüche 1 bis 7,
dadurch gekennzeichnet,
daß die Küvette (10) in getrenntem Zustand von Kapillarhalter (11) mittels eines lösbaren Küvettenverschlusses (18) verschlossen ist.

10. Probenabnahmegerät nach Anspruch 9,
dadurch gekennzeichnet,
daß der Küvettenverschluß (18) als Druckkappe verwendbar ist und in eine erste, den Fortsatz (14) nur teilweise übergreifenden Stellung aufsetzbar und in eine zweite, den Fortsatz (14) vollständig übergreifende Stellung aufschiebbar ausgebildet ist.

11. Probenabnahmegerät nach Anspruch 10,
dadurch gekennzeichnet,
daß das Innenvolumen in dem als Druckkappe ausgebildeten Küvettenverschluß (18) derart bemessen ist, daß beim Aufsetzen des Küvettenverschlusses (18) auf den Fortsatz (14) und anschließenden vollständigen Aufschieben die Probenflüssigkeit aus der Kapillare (16) gerade herausgedrückt wird.

12. Probenabnahmegerät nach einem der Ansprüche 1 bis 11,
dadurch gekennzeichnet,
daß die Kapillare (16) eine End-zu-End-Kapillare ist.

## Claims

1. Sample collection device (100) for the collection of capillary blood on the patient direct or for the collection of a sample from a liquid with the aid of the capillary (16), which sample collection device possesses one capillary and one cuvet, in which case the capillary (16), for the reception of sample liquid to be analyzed, is connectable with the cuvet (10) with the aid of a mounting means (110) and the cuvet (10), in the form of a mounting means (110), is provided with a capillary holder (11) retaining the capillaries (16),
**characterized in that**
the capillary holder (11) possesses a stopper-like portion (12) which, when in use, projects into the cuvet (10), while the capillary holder (11) possesses a circumferential rim (13) which is larger than the cuvet circumference, and in that, on the side pointing away from the cuvet (10), in the center of the capillary holder (11), an extension (14) is provided, through which, as also through the stopper-like portion (12), a through bore (15) extends, into which the capillary (16) is inserted and in that a pressure cap (17) is provided which can be slipped on to the extension (14), the volume (23) in the pressure cap (17) being dimensioned in such a way that, when the pressure cap (17) is fitted on the extension (14) and by the subsequent complete sliding on, the sample liquid is pressed out straight from the capillary (16), in that, in a reagent fluid (19) in the cuvet (10), an anticoagulant, such as e.g. a citrate buffer, is contained and in that, in the cuvet (10), a stirring member (20) is comprised.

2. Sample collection device according to Claim 1,
**characterized in that**
the capillary (16) is mounted in the capillary holder (11) so as to be longitudinally displaceable.

3. Sample collection device according to either Claim 1 or 2,
**characterized in that**
the cuvet (10) is closed with the capillary holder (11).

4. Sample collection device according to Claim 1,
**characterized in that**
the pressure cap (17) is designed so that the same can be fitted assuming a first position, wherein it engages only partly over the extension (14) and so that it can be fitted while assuming a second position, wherein is engages completely over the extension (14).

5. Sample collection device according to any of Claims 1 to 4,
**characterized in that**
the through bore (15) in the capillary holder (11) is shorter than the length of the capillary (16), more particularly 20% to 30% shorter.

6. Sample collection device according to Claim 1,
**characterized in that,**
within the bearing area of tee capillary holder (11) on the cuvet (10) and/or within the contact area of the circumferential rim (13) of the capillary holder (11) in the direction of the cuvet (10), grooves are formed which permit a pressure equalization between the interior (21) of the cuvet (10) and the environment.

7. Sample collection device according to either Claim 1 or 6,
**characterized in that**
the circumferential rim (13) of the capillary holder (11) in the direction of the cuvet (10) is turned down on the exterior (22) of the latter.

8. Sample collection device according to any of Claims 1 to 7,
**characterized in that**,
the cuvet (10), in the state separated from the capillary holder (11), is sealed by means of an evaporation-proof foil.

9. Sample collection device according to any of Claims 1 to 7,
**characterized in that,**
the cuvet (10), in the state separated from the capillary holder (11), is ealed by means of a detachable cuvet closure (18).

10. Sample collection device according to Claim 9,
**characterized in that**
the cuvet closure (18) can be utilized as pressure cap and can be fitted so as to assume a first position, wherein it engages only in part over the extension (14) and can also be fitted so as to assume a second position, wherein it engages completely over the extension (14).

11. Sample collection device according to Claim 10,
**characterized in that**
the internal volume in the cuvet closure (18) constructed in the form of a pressure cap is dimensioned in such a way that, when the cuvet closure (18) is fitted on to the extension (14) and when the same is subsequently slid on completely, the sample liquid is presseed out straight from the capillary (16).

12. Sample collection device according to any of Claims 1 to 11,
**characterized in that**
the capillary (16) is an end-to-end capillary.

## Revendications

1. Appareil de prélèvement d'échantillons (100) pour le prélèvement de sang capillaire directement sur le patient ou pour le prélèvement d'un échantillon d'un liquide au moyen d'un tube capillaire (16), lequel appareil de prélèvement d'échantillons présente un tube capillaire et une cuvette, le tube capillaire (16) pouvant être relié, pour recueillir du liquide d'échantillon à analyser, au moyen d'un dispositif de support (110) à la cuvette (10) et la cuvette (10) présentant comme dispositif de support (110) un support de tube capillaire (11) qui maintient le tube capillaire (16),
caractérisé en ce
que le support de tube capillaire (11) présente une zone du type bouchon (12) qui, lors de l'utilisation, entre dans la cuvette (10), le support de tube capillaire (11) possédant un bord périphérique (13) qui est plus grand que la périphérie de la cuvette et que, sur le côté détourné de la cuvette (10) au centre du support de tube capillaire (11) il est prévu un prolongement (14) à travers lequel s'étend, tout comme aussi à travers la zone du type bouchon (12), une forure de passage (15) dans laquelle le tube capillaire (16) est enfoncé et qu'un capuchon de pression (17) est prévu qui peut être emboîté sur le prolongement (14), le volume (23) dans le capuchon de pression (17) étant dimensionné tel que, lors de la pose du capuchon de pression (17) sur le prolongement (14) et lors de l'action de l'enfiler complètement qui suit, le liquide de l'échantillon est tout juste exprimé du tube capillaire (16), qu'un anticoagulant, comme par exemple un tampon de citrate, est contenu dans un liquide réactif (19) dans la cuvette (10) et qu'un corps agitateur (20) est contenu dans la cuvette (10).

2. Appareil de prélèvement d'échantillons selon la revendication 1,
caractérisé en ce
que le tube capillaire (16) est maintenu en étant coulissant dans le sens longitudinal dans le support de tube capillaire (11).

3. Appareil de prélèvement d'échantillons selon la revendication 1 ou 2,
caractérisé en ce
que la cuvette (10) est fermée avec le support de tube capillaire (11).

4. Appareil de prélèvement d'échantillons selon la revendication 1,
caractérisé en ce
que le capuchon de pression (17) est configuré en pouvant être posé dans une première position qui ne recouvre que partiellement le prolongement (14) et en pouvant être enfilé dans une seconde position qui recouvre complètement le prolongement (14).

5. Appareil de prélèvement d'échantillons selon l'une des revendications 1 à 4,
caractérisé en ce
que la forure de passage (15) dans le support de tube capillaire (11) est plus courte que la longueur du tube capillaire (16), en particulier de 20 % à 30 %.

6. Appareil de prélèvement d'échantillons selon la revendication 1,
caractérisé en ce
que des rainures sont ménagées dans la zone d'appui du support du tube capillaire (11) sur le cuvette (10) et/ou dans la zone d'appui du bord périphérique (13) du support du tube capillaire (11) vers la cuvette (10), rainures qui permettent une compensation de pression entre l'espace intérieur (21) de la cuvette (10) et l'environnement.

7. Appareil de prélèvement d'échantillons selon la revendication 1 ou 6,
caractérisé en ce
que le bord périphérique (13) du support de tube capillaire (11) est rabaissé on direction de la cuvette (10) sur son côté extérieur (22).

8. Appareil de prélèvement d'échantillons selon l'une des revendications 1 à 7,
caractérisé en ce
que la cuvette (10), à l'état séparé du support de tube capillaire (11), est fermée par une feuille étanche à l'évaporation.

9. Appareil de prélèvement d'échantillons selon l'une des revendications 1 à 7,
caractérisé en ce
que la cuvette (10), à l'état séparé du support de tube capillaire (11), est fermée au moyen d'une fermeture de cuvette amovible (18).

10. Appareil de prélèvement d'échantillons selon la revendication 9,
caractérisé en ce
que la fermeture de la cuvette (18) peut être utilisée comme capuchon de pression et est configurée en pouvant être posée dans une première position qui ne recouvre que partiellement le prolongement (14) et en pouvant être enfilée dans une seconde position qui recouvre complètement le prolongement (14).

11. Appareil de prélèvement d'échantillons salon la revendication 10,
caractérisé en ce
que le volume intérieur dans la fermeture de cuvette (18) configurée comme capuchon de pression est dimensionné tel que, lors de la pose de la fermeture de cuvette (18) sur le prolongement (14) et lors de l'action de l'enfiler complètement qui suit, le liquide de l'échantillon est exprimé tout juste du tube capillaire (16).

12. Appareil de prélèvement d'échantillons selon l'une des revendications 1 à 11,
caractérisé en ce
que le tube capillaire (16) est un tube capillaire bout à bout.
